# EUROPÄISCHE PATENTSCHRIFT

(11) **EP 0 342 499 B1**
(45) Veröffentlichungstag und Bekanntmachung des Hinweises auf die Patenterteilung: **19.01.1994**
(21) Anmeldenummer: 89108379.2
(22) Anmeldetag: 10.05.1989
(51) Int. Cl.: A61L 2/18

(54) **Verfahren zur Reinigung und Desinfektion von hitze- und korrosionsempfindlichen medizinischen Geräten, insbesondere von Endoskopen und Mittel zur Durchführung des Verfahrens**
Method for cleaning and disinfecting heat and corrosion sensitive medical devices, especially endoscopes, and product for carrying out the method
Procédé de nettoyage et de désinfection d'articles médicaux sensibles à la chaleur et la corrosion, en particulier d'endoscopes et produit pour mettre en oeuvre ce procédé

(30) Priorität: 17.05.1988 DE 3816734
(43) Veröffentlichungstag der Anmeldung: 23.11.1989
(73) Patentinhaber: Henkel Kommanditgesellschaft auf Aktien, 40191 Düsseldorf (DE)
(72) Erfinder: Disch, Karlheinz, Dr., D-5657 Haan (DE); Hachmann, Klaus, Dr., D-4010 Hilden (DE); Bansemir, Klaus, Dr., D-4018 Langenfeld (DE)

(56) Entgegenhaltungen:
- EP-A- 0 118 933
- EP-A- 0 268 227
- CH-A- 434 574
- DE-A- 3 327 466
- US-A- 3 697 222

## Beschreibung

Bei der medizinischen Diagnostik und Therapie werden chirurgische Eingriffe in steigendem Maß durch den Einsatz von Endoskopen ersetzt. Diese Entwicklung ist vor allem dadurch möglich geworden, daß seit einiger Zeit flexible Glasfiberendoskope zur Verfügung stehen. Bei der bestimmungsgemäßen Verwendung werden die Endoskope massiv mit Mikroorganismen infiziert, die sich in den Körperhöhlen, auf der Schleimhaut und im Blut befinden. Benutzte Endoskope müssen deshalb nach jedem Einsatz gründlich gereinigt und desinfiziert werden.

Glasfiberendoskope stellen außerordentlich komplizierte Präzisionsinstrumente dar, die bewegliche Teile besitzen und aus einer Vielzahl von Materialien gefertigt sind. Ihre Reinigung und Desinfektion ist aus einer Reihe von Gründen höchst problematisch. So sind jeweils nicht nur die außenliegenden Oberflächen des Instrumentes sondern auch die im Inneren vorhandenen englumigen Kanäle zu reinigen und zu desinfizieren. Mit Rücksicht auf die empfindlichen Materialien soll die Reinigung und Desinfektion so durchgeführt werden, daß auf den behandelten Oberflächen des Instrumentes keine Rückstände der verwendeten Mittel verbleiben. Die bei medizinischen Instrumenten übliche und höchst wirksame thermische Sterilisation kann hier nicht angewendet werden, da die Endoskope zum Teil aus temperaturempfindlichen Materialien gefertigt sind. Weiterhin ist zu berücksichtigen, daß eine Reihe von vorhandenen Metallteilen korrosionsanfällig ist. Schließlich soll sich die Reinigung und Desinfektion der Endoskope in kurzer Zeit durchführen lassen, damit die Instrumente möglichst schnell für die Behandlung der nächsten Patienten wieder zur Verfügung stehen. Erst vor wenigen Jahren ist es den Herstellern von Glasfiberendoskopen gelungen, Instrumente zu entwickeln, die vollständig in Reinigungs- und Desinfektionsbäder eingelegt werden können und Temperaturen von bis zu 70 °C unbeschadet aushalten. Vergleichbare Verhältnisse liegen bei einer Reihe von anderen medizinischen Geräten vor, die ebenfalls aus temperaturempfindlichen und/oder korrosionsanfälligen Materialien angefertigt sind.

Aus der DE-PS 33 27 466 ist ein Verfahren zum Reinigen von Gebrauchsgegenständen aus dem Bereich der Medizin und der Krankenpflege bekannt, bei dem die Gebrauchsgegenstände in einem geschlossenen System bei Temperaturen von höchstens 70 °C in einer Trägerflüssigkeit (Wasser) mit einem üblichen Reinigungsmittel behandelt werden und anschließend ein Desinfektionsmittel zusätzlich in die Trägerflüssigkeit eingegeben wird. Dabei wird als Desinfektionsmittel eine Mischung aus Glutaraldehyd und/oder Bernsteinsäuredialdehyd mit einem Salicylat und einem Polyethylenglykol eingesetzt. Bei der Durchführung des Verfahrens sind für die Reinigungsmittelbehandlung und die Desinfektionsmittelbehandlung separate Temperatur-Haltezeiten vorgesehen. Für die Reinigungsbehandlung werden alkalische Reinigungsmittelzusammensetzungen empfohlen. Dieses Verfahren hat sich für die Reinigung und Desinfektion von Endoskopen als nicht geeignet erwiesen, vor allem deshalb, weil es bereits nach einer begrenzten Zahl von Verfahrenszyklen zu Korrosionserscheinungen an den Metallteilen der Endoskope führt.

Der Erfindung lag die Aufgabe zugrunde, ein Verfahren zu entwickeln, das durch eine Kombination von thermischer und chemischer Behandlung eine zuverlässige Reinigung und Desinfektion der von hitze- und korrosionsempfindlichen medizinischen Geräten, insbesondere von Endoskopen, in kurzer Zeit ermöglicht und auch bei Daueranwendung keine Schädigung der behandelten Instrumente zur Folge hat. Dieses Verfahren sollte so ausgelegt sein, daß es gegebenenfalls in einer automatisch arbeitenden Spülmaschine durchgeführt werden kann. Ferner sollte das Verfahren so gestaltet werden können, daß die gebrauchten Reinigungs- und Desinfektionslösungen steril sind, so daß sie ohne Bedenken dem normalen Abwasser beigemischt werden können. Diese Aufgabe wird durch das nachstehend beschriebene Verfahren gelöst.

Gegenstand der Erfindung ist ein Verfahren zur Reinigung und Desinfektion von hitze- und korrosionsempfindlichen medizinischen Geräten, insbesondere von Endoskopen, mit Hilfe von wässrigen Reinigungs- und Desinfektionsmittellösungen, das dadurch gekennzeichnet ist, daß man nacheinander
a) die zu behandelnden Oberflächen mit einer Reinigung- und Desinfektionsmittellösung, die
   - mindestens ein schaumarmes nichtionogenes Tensid,
   - mindestens ein proteolytisches Enzym,
   - mindestens einen Komplexbildner,
   - mindestens einen Aldehyd aus der aus Formaldehyd und aliphatischen Dialdehyden mit 2 bis 8 Kohlenstoffatomen bestehenden Gruppe und
   - gegebenenfalls weitere übliche Reinigungs- und Desinfektionsmittelbestandteile
   enthält und einen pH-Wert von 6 bis 8 besitzt, in Kontakt bringt, die Reinigungslösung auf 55 bis 65 °C erhitzt, 1 bis 15 Minuten lang auf dieser Temperatur hält und anschließend abtrennt,
b) die zu behandelnden Oberflächen mindestens zweimal mit Wasser spült, wobei man zumindest im letzten Spülgang das Wasser auf 55 bis 65 °C erhitzt, und
c) mit sterilisierter Heißluft bei 40 bis 60 °C trocknet,
wobei man in den Schritten a) und b) Wasser mit einer Härte von 3 bis 8 °d einsetzt.

Es hat sich als zweckmäßig erwiesen, im Schritt a) eine Reinigungs- und Desinfektionsmittellösung einzusetzten, die
0,1 bis 1,0 g/l schaumarmes Tensid,
0,03 bis 0,3 AE/l proteolytisches Enzym,
0,02 bis 0,3 g/l Komplexbildner und
0,5 bis 5 g/l Aldehyd
enthält (AE = Anson-Einheiten).

Als schaumarme nichtionogene Tenside, die sich für die Verwendung in der Reinigungs- und Desinfektionsmittellösung des Schrittes a) eignen, sind vor allem Alkylenoxidaddukte zu nennen, wie sie durch Anlagerung von 3 bis 30 Mol Ethylenoxid und/oder Propylenoxyd an aliphatische Polyole mit 2 bis 6 Hydroxylgruppen und 2 bis 12 Kohlenstoffatomen sowie an Fettalkohole, Fettsäuren, Fettamine oder Alkylphenole mit jeweils 8 bis 18 Kohlenstoffatomen erhalten werden können, wobei die endständigen Hydroxylgruppen dieser Polyglykoletherderivate auch verethert, verestert oder acetalisiert sein können. Besonders geeignet sind Anlagerungsprodukte von 3 bis 15 Mol Ethylenoxyd an gesättigte und ungesättigte Fettalkohole mit 8 bis 18 Kohlenstoffatomen, Anlagerungsprodukte von 3 bis 5 Mol Ethylenoxid und 3 bis 6 Mol Propylenoxid an gesättigte und ungesättigte Fettalkohole mit 8 bis 18 Kohlenstoffatomen, wobei diese gemischten Alkylenoxidaddukte sowohl in Random- als auch im Blockpolymerisationsverfahren hergestellt sein können, sowie Etherderivate der vorgenannten Fettalkoholalkylenglykolether, in denen die endständigen Hydroxylgruppen mit einem geradkettigen oder verzweigten gesättigten aliphatischen Alkohol mit 4 bis 8 Kohlenstoffatomen verethert sind. Von besonderer Bedeutung sind hier Polyethylenglykolether der Formel I,

R¹ - O - (CH₂CH₂O)ₙ - R² (I),

in der R¹ einen geradkettigen oder verzweigten Alkyl- oder Alkenylrest mit 8 bis 18 Kohlenstoffatomen, R² einen geradkettigen oder verzweigten Alkylrest mit 4 bis 8 Kohlenstoffatomen und n eine Zahl von 7 bis 12 bedeutet, wobei hier Polyethylenglykolether der Formel I besonders bevorzugt sind, in der R¹ für einen aus einem gehärteten oder ungehärteten Talgfettalkohol stammendes Gemisch aus Alkyl- und/oder Alkenylresten mit 12 bis 18 Kohlenstoffatomen und R² für einen n-Butylrest steht, während n eine Zahl von 9 bis 10 bedeutet.

Als proteolytische Enzyme kommen für die Reinigungs- und Desinfektionsmittellösung des Schrittes a) insbesondere aus Bakterienstämmen gewonnene Proteasen in Betracht. Geeignet sind z.B. die aus Bacillus subtilis, Bacillus licheniformis und Streptomyces griseus gewonnenen Enzyme. Entsprechende handelsübliche Präparate liegen entweder in Form von Lösungen des Enzyms in einem Gemisch aus Wasser und einem organischen Lösungsmittel, beispielsweise 1,2-Propandiol, oder als feste Granulate vor. Diese Handelsformen enthalten in der Regel wasserlösliche Calciumsalze als Potenzierungs- und Stabilisierungsmittel. Feste Präparate sind mittels Stellmittel, beispielsweise Natriumsulfat, Natriumchlorid, Alkaliphosphat oder Alkalipolyphosphat auf einen bestimmten Aktivitätsgrad eingestellt.

Als Komplexbildner kann die Reinigungslösung des Schrittes a) beispielsweise Alkalisalze der Nitrilotriessigsäure, Ethylendiaminotetraessigsäure, 1-Hydroxyethan-1,1-diphosphonsäure, Aminotris-(methylenphosphonsäure), Ethylendiamiotetrakis-(methylenphosphonsäure), Phosphonobutantricarbonsäure, Weinsäure, Citronensäure und Glukonsäure enthalten. Besonders bevorzugt ist hier Phosphonobutantricarbonsäure.

Beispiele für die in der Reinigungs- und Desinfektionsmittellösung des Schrittes a) vorhandenen aliphatischen Dialdehyde mit 2 bis 8 Kohlenstoffatomen sind Glyoxal, Malonaldehyd, Succinaldehyd und Glutaraldehyd, wobei Glutaraldehyd bevorzugt ist.

Bei der Durchführung des erfindungsgemäßen Verfahrens wird prinzipiell Wasser mit einer Härte im Bereich von 3 bis 8 °d eingesetzt. Dies gilt sowohl für das Ansetzen der Reinigungslösung als auch für die Spülgänge. Die Einstellung der vorgenannten Härtegrade erfolgt zweckmäßigerweise dadurch, daß man Leitungswasser zumindest anteilweise über einen Kationenaustauscher leitet, der die härtebildenden Kationen aus dem Wasser entfernt. Der Durchgang durch den Kationenaustauscher hat eine Verschiebung des pH-Wertes in den alkalischen Bereich zur Folge. Aus diesem Grund ist es erforderlich, daß der pH-Wert der Reinigungs- und Desinfektionsmittellösung des Schrittes a) auf den angegebenen Bereich von 6 bis 8 eingestellt wird. Aus Gründen der Lagerbeständigkeit hat es sich als zweckmäßig erwiesen, für die Herstellung der Reinigungs- und Desinfektionsmittellösung des Schrittes a) jeweils getrennte Reinigungsmittel- und Desinfektionsmittelkonzentrate bereitzustellen. Derartige Konzentrate lassen sich so formulieren, daß sie beim Verdünnen mit dem kationenaustauscherbehandelten Leitungswasser Lösungen mit einem pH-Wert im geforderten Bereich ergeben. Das für die Spülgänge verwendete Wasser kann gegebenenfalls mit Hilfe von physiologisch unbedenklichen organischen Säuren, beispielsweise mit Essigsäure, Weinsäure, Milchsäure, Äpfelsäure, Citronensäure oder auf einen pH-Wert im Bereich von 6 bis 8 eingestellt werden, wenn der pH-Wert des enthärteten Wassers 8,5 wesentlich übersteigt.

Während der Behandlung der Endoskope im Schritt a) des erfindungsgemäßen Verfahrens können die Endoskope gleichzeitig - zur Verstärkung der reinigenden bzw. der desinfizierenden Wirkung -der Einwirkung von Ultraschall ausgesetzt werden.

Zum Trocknen der Endoskope im Schritt d) verwendet man vorzugsweise Luft, die vor dem Erhitzen durch Ansaugen über einen Mikrofilter sterilisiert wurde.

Die aus dem Schritt a) resultierende gebrauchte Reinigungs- und Desinfektionsmittellösung kann ohne weitere Nachbehandlung in die Kanalisation abgelassen werden.

Die praktische Durchführung des erfindungsgemäßen Verfahrens kann beispielsweise in entsprechend dimensionierten, verschließbaren Edelstahlbehältern erfolgen, die beheizbar sind und mit einer Einrichtung zum Umpumpen der verschiedenen Flüssigkeiten und der zur Trocknung verwendeten Heißluft durch die zu reinigenden Kanäle der Endoskope versehen sind. Weiterhin sollen Zu- und Ableitungen für die Reinigungs- und die Desinfektionslösung und das Spülwasser sowie für die zur Trocknung der Instrumente benötigte Heißluft vorhanden sein. Es ist von Vorteil, wenn die zu behandelnden Endoskope in ein in den Edelstahlbehälter passendes Gestell eingelegt werden können. Für die Durchführung der einzelnen Schritte des erfindungsgemäßen Verfahrens wird der Behälter jeweils mit so viel Flüssigkeit beschickt, daß die Endoskope vollständig damit bedeckt sind. Die jeweils vorhandene Flüssigkeit wird ständig mit einer ausreichenden Geschwindigkeit durch die Kanäle des Endoskops umgepumpt. Beim Ablassen der Behandlungsflüssigkeiten ist dafür Sorge zu tragen, daß auch die jeweils in den Kanälen vorhandene Flüssigkeit entfernt wird.

Für die Durchführung des erfindungsgemäßen Verfahrens eignen sich insbesondere automatisch arbeitende Spülmaschinen, wie sie für die Reinigung von Laborgeräten und medizinischen Instrumenten bekannt und üblich sind, unter der Voraussetzung, daß sie die erforderlichen Zusatzeinrichtungen aufweisen, beispielsweise eine Einrichtung, mit deren Hilfe die Flüssigkeiten durch die Kanäle der Endoskope bewegt werden können. Die äußeren Flächen der Endoskope werden hier nicht durch Einlegen in die Flüssigkeiten mit diesen in Kontakt gebracht, sondern durch laufendes Besprühen.

Für die Verwendung im Schritt a) werden zweckmäßigerweise beständige, lagerfähige Reinigungsmittel- und Desinfektionsmittelkonzentrate bereitgestellt, die außer den im Vorstehenden bereits genannten Wirkstoffen noch weitere in solchen Konzentraten üblichen Bestandteile enthalten.

Ein wäßriges Reinigungskonzentrat für die Herstellung der in Schritt a) verwendeten Reinigungs- und Desinfektionsmittellösung kann beispielsweise
5 bis 10 Gew.-% schaumarmes nichtionogenes Tensid,
7,1 bis 77 AE/l proteolytisches Enzym,
20 bis 60 Gew.-% Enzymstabilisator,
1 bis 5 Gew.-% Konfektionierhilfsmittel und
0,05 bis 0,5 Gew.-% Konservierungsmittel
enthalten. Der pH-Wert des Konzentrates wird mit Säure, Base oder einem Säure-Basengemisch auf 4 bis 6 eingestellt.

Als Enzymstabilisator kann das wässrige Reinigungsmittelkonzentrat beispielsweise Triethanolamin, Morpholin, alpha-Pyrrolidon, Ethylenglykol, Propylenglykol, Glycerin, wasserlösliche Calciumsalze oder Gemische dieser Verbindungen enthalten. Vorzugsweise wird hier Glycerin und/oder Propylenglykol als Enzymstabilisator eingesetzt.

Für das wässrige Reinigungsmittelkonzentrat geeignete Konfektionierhilfsmittel (Lösungsvermittler) sind beispielsweise Natriumcumolsulfonat, Natriumtoluolsulfonat, Natriumxylolsulfonat, Harnstoff, Polyethylenglykole, Methylacetamid und Fettalkohole, wie Cetylalkohol, bevorzugt wird Natriumcumolsulfonat als Konfektionierhilfsmittel eingesetzt.

Reinigungsmittelkonzentrate der beschriebenen Art sind gegen Mikrobenbefall anfällig. Insbesondere wird bei nichtkonservierten Zusammensetzungen leicht Pilzwachstum beobachtet. Aus diesem Grund setzt man den Konzentraten wirksame Mengen Konservierungsmittel zu. Geeignete Konservierungsmittel sind beispielsweise p-Hydroxybenzoesäuremethylester, 5-Brom-5-nitro-1,3-dioxan, Glutaraldehyd, Salicylsäure, 0-2-Naphthyl-m-N-dimethylthiocarbanilat, 5-Chlor-5-methyl-4-isothiazolin-3-on, 2-Methyl-4-isothiazolin-3-on, Benzisothiazolin-3-on und Gemische der beiden zuletzt genannten Verbindungen. Vorzugsweise wird p-Hydroxybenzoesäuremethylester als Konservierungs- mittel eingesetzt.

Für die in dem wässrigen Reinigungsmittelkonzentrat enthaltenen Bestandteile schaumarmes nichtionogenes Tensid und proteolytisches Enzym gelten die in der vorstehenden Beschreibung des erfindungsgemäßen Verfahrens enthaltenen Angaben in vollem Umfang.

Ein wässriges Desinfektionsmittelkonzentrat für die Verwendung in Schritt a) kann beispielsweise
10 bis 40 Gew.-% Aldehyd aus der aus Formaldehyd und aliphatischen Dialdhyden mit 2 bis 8 Kohlenstoffatomen bestehenden Gruppe
0,5 bis 2 Gew.-% Komplexbildner und
7 bis 15 Gew.-% Konfektionierhilfsmittel
enthalten. Der pH-Wert des Konzentrates wird mit Säure, Base oder einem Säure-Basengemisch auf 3 bis 5 eingestellt.

Als Konfektionierhilfsmittel für das Desinfektionsmittelkonzentrat kommen insbesondere niedere aliphatische Alkohole wie Ethanol, n-Propanol und Isopropanol sowie Ethylenglykol und Triacetin in Betracht. Vorzugsweise wird Ethanol als Konfektionierhilfsmittel verwendet.

Bezüglich der in dem wässrigen Desinfektionsmittelkonzentrat enthaltenen Bestandteile aliphatischer Dialdehyd und Komplexbildner gelten wiederum die in der vorstehenden Beschreibung des erfindungsgemäßen Verfahrens enthaltenen Angaben in vollem Umfang.

### Beispiel

Durch mechanisches Vereinigen der Einzelbestandteile wurden Konzentrate der folgenden Zusammensetzung (GT = Gewichtsteile) hergestellt:

### Reinigungsmittelkonzentrat

8 GT n-Butylether eines Anlagerungsproduktes von 9,5 Mol Ethylenoxid an 1 Mol gehärteten Talgfettsalkohol (Formel I: R¹ = C₁₂₋₁₈-Alkyl; R² = C₄-Alkyl; n = 9,5)
1 GT proteolytisches Enzym (Alcalase ^{R}, Hersteller: Novo Industri A/S, Bagsvaerd, Dänemark; 2,5 AE/g)
6 GT Glycerin
50 GT 1,2-Propylenglykol
2 Gt Citronensäure
3 GT Natriumcumolsulfonat
0,1 GT p-Hydroxybenzoesäuremethylester
ad 100 GT Wasser
Das Gemisch wurde mit 37 gew.-%iger Natriumhydroxydlösung auf pH 5 eingestellt.

### Desinfektionsmittelkonzentrat

20 GT Glutaraldehyd
1 GT Phosphonobutantricarbonsäure
8 GT Ethanol
ad 100 GT Wasser
Das Gemisch wurde mit 50 gew.-%iger Natriumhydroxydlösung auf pH 4 eingestellt.

Die Reinigung und Desinfektion der Endoskope erfolgte in einem verschließbaren, mit einer Heizung versehenen Edelstahlgefäß (Durchmesser ca. 60 cm; Höhe ca. 65 cm), das Zu- und Ableitungen für die Reinigungs- und Desinfektionsmittellösung und das in den Spülgängen benutzte Wasser sowie für die zur Trocknung der Instrumente benötigte Heißluft besaß. Die Apparatur war mit einer Umlaufpumpe versehen, mit deren Hilfe die jeweils vorhandene Flüssigkeit durch die Kanäle der Fiberendoskope gepumpt werden konnte.

Die Versuche wurden mit einem handelsüblichen Gastroskop durchgeführt.

Zum Ansetzen der Reinigungs- und der Desinfektionsmittellösung wurde Wasser verwendet, das mit Hilfe eines Kationenaustauschers auf eine Härte von 5° d eingestellt war. Dasselbe Wasser wurde zur Durchführung der Spülgänge eingesetzt, nachdem es mit Hilfe von Milchsäure auf pH 7 eingestellt war.

Durch Verdünnen entsprechender Anteile Reinigungsmittelkonzentrat und Desinfektionsmittelkonzentrat wurde eine anwendugsfertige Reinigungs- und Desinfektionsmittellösung hergestellt, die 0,45 g/l Tensid, 0,06 g/l Enzym, 2,4 g/l Glutaraldehyd und 0,12 g/l Phosphonobutantricarbonsäure enthielt.

Die für die Trocknung eingesetzte Luft wurde über ein Mikrofilter angesaugt und vor dem Einleiten in das Edelstahlgefäß durch eine Heizstrecke geleitet, wo sie auf 60 °C erwärmt wurde.

Bei der Durchführung des Reinigungsverfahrens wurde das Endoskop in einem Drahtkorb in den Edelstahlbehälter eingelegt. Die Kanäle des Endoskops wurden an die Umlaufpumpe angeschlossen. In den einzelnen Schritten des Verfahrens wurde dem Edelstahlbehälter jeweils soviel Flüssigkeit zugeführt, daß das Endoskop ganz bedeckt war. Während der einzelnen Verfahrensschritte wurde die vorhandene Flüssigkeit laufend durch die Kanäle des Endoskops umgepumpt.

Nach dem Befüllen des Edelstahlgefäßes mit Reinigungs- und Desinfektionsmittellösung wurde diese auf 60 °C erhitzt und 10 Minuten lang auf dieser Temperatur gehalten. Nach dem Abtrennen der Reinigungs- und Desinfektionsmittellösung wurde zweimal mit kaltem Wasser gespült. Anschließend wurde das Edelstahlgefäß erneut mit Wasser gefüllt, das nun auf 60 °C erhitzt und danach abgezogen wurde. Zur Trocknung des Endoskops wurde schließlich 5 Minuten lang sterile Heißluft eingeleitet.

Zur Prüfung der bei dem erfindungsgemäßen Verfahren erzielten desinfizierenden Wirkung wurden die Kanäle des Endoskops gesondert mit Keimsuspensionen kontaminiert, die folgende Keime enthielten:
1) ca. 10⁹ Keime/ml Staphylococcus aureus
2) ca. 10⁹ Keime/ml Pseudomonas aeroginosa
3) ca. 10⁷ Keime/ml Candida albicans
Zur Simulierung praxisnaher Bedingungen enthielten die Keimsuspensionen einen Zusatz von 20 Gewichtsprozent defibrinierten Hammelblutes.

Bei der Kontaminierung wurden die Kanäle des Endoskops mit den Keimsuspensionen gefüllt. Nach kurzem Stehenlassen wurden die Keimsuspensionen wieder abgelassen. Jeweils eine Stunde nach der Kontamination wurde das Endoskop erfindungsgemäß gereinigt und desinfiziert. Anschließend wurden 0,5 l einer Lösung, die 3 Gewichtsprozent Tween 80, 0,3 Gewichtsprozent Lecithin, 0,1 Gewichtsprozent Histidin, 0,1 Gewichtsprozent Trypton und 0,05 Gewichtsprozent Natriumchlorid enthielt, durch die Kanäle des Endoskops gesaugt. Proben von jeweils 1 ml dieser Lösung wurden auf Aggarplatten überimpft, die anschließend mindestens 48 Stunden bei 37 °C beziehungsweise mindestens 72 Stunden bei 35 °C bebrütet und danach auf vorhandenes Keimwachstum geprüft wurden.

Es wurde festgestellt, daß bei der Durchführung des erfindungsgemäßen Verfahrens in allen Fällen die geforderte Keimfreiheit erzielt worden war.

In weiteren Versuchen, die unter den oben beschriebenen Verfahrensbedingungen durchgeführt wurden, konnte gezeigt werden, daß das erfindungsgemäße Verfahren mit Erfolg auch bei Kontaminationen durch Enteroviren wie Polio- und Pavovaviren sowie durch Hepatitis-B-viren eingesetzt werden kann.

## Patentansprüche

1. Verfahren zur Reinigung und Desinfektion von hitze- und korrosionsempfindlichen medizinischen Geräten, insbesondere von Endoskopen, mit Hilfe von wässrigen Reinigungs- und Desinfektionsmittellösungen, dadurch gekennzeichnet, daß man nacheinander
a) die zu behandelnden Oberflächen der Endoskope mit einer Reinigungs- und Desinfektionsmittellösung, die
- mindestens ein schaumarmes nichtionogenes Tensid,
- mindestens ein proteolytisches Enzym,
- mindestens einen Komplexbildner,
- mindestens einen Aldehyd aus der aus Formaldehyd und aliphatischen Dialdehyden mit 2 bis 8 Kohlenstoffatomen bestehenden Gruppe und
- gegebenenfalls weitere übliche Reinigungs- und Desinfektionsmittelbestandteile
enthält und einen pH-Wert von 6 bis 8 besitzt, in Kontakt bringt, die Reinigungslösung auf 55 bis 65 °C erhitzt, 1 bis 15 Minuten lang auf dieser Temperatur hält und anschließend abtrennt,
b) die zu behandelnden Oberflächen mindestens zweimal mit Wasser spült, wobei man zumindest im letzten Spülgang das Wasser auf 55 bis 65 °C erhitzt, und
c) mit sterisilierter Heißluft bei 40 bis 60 °C trocknet,
wobei man in den Schritten a) und b) Wasser mit einer Härte von 3 bis 8 °d einsetzt.

2. Verfahren nach Anspruch 1, dadurch gekennzeichnet, daß die Reinigungs- und Desinfektionsmittellösung im Schritt a)
0,1 bis 1,0 g/l schaumarmes Tensid,
0,03 bis 0,3 AE/l proteolytisches Enzym,
0,02 bis 0,3 g/l Komplexbildner und
0,5 bis 5 g/l Aldehyd
enthält (AE = Anson-Eiheiten).

3. Verfahren nach den Ansprüchen 1 und 2, dadurch gekennzeichnet, daß die Reinigungs- und Desinfektionsmittellösung in Schritt a) als schaumarmes nichtionogenes Tensid ein Alkylenoxidaddukt enthält, das durch Anlagerung von 3 bis 30 Mol Ethylenoxid und/oder Propylenoxid an aliphatische Polyole mit 2 bis 6 Hydroxylgruppen und 2 bis 12 Kohlenstoffatome sowie an Fettalkohole, Fettsäuren, Fettamine oder Alkylphenole mit jeweils 8 bis 18 Kohlenstoffatomen erhältlich ist, wobei die endständigen Hydroxylgruppen dieser Polyglykoletherderivate auch verethert, verestert oder acetalisiert sein können.

4. Verfahren nach den Ansprüchen 1 bis 3, dadurch gekennzeichnet, daß die Reinigungs- und Desinfektionsmittellösung in Schritt a) ein schaumarmes nichtionogenes Tensid aus der Gruppe Anlagerungsprodukte von 3 bis 15 Mol Ethylenoxid an gesättigte und ungesättigte Fettalkohole mit 8 bis 18 Kohlenstoffatomen, Anlagerungsprodukte von 3 bis 5 Mol Ethylenoxid und 3 bis 6 Mol Propylenoxid an gesättigte und ungesättigte Fettalkohole mit 8 bis 18 Kohlenstoffatomen und Etherderivate dieser Fettalkoholpolyalkylenglykolether, in denen die endständigen Hydroxylgruppen mit einem geradkettigen oder verzweigten gesättigten aliphatischen Alkohol mit 4 bis 8 Kohlenstoffatomen verethert sind, enthält.

5. Verfahren nach Anspruch 4, dadurch gekennzeichnet, daß die Reinigungs- und Desinfektionsmittellösung in Schritt a) als schaumarmes Tensid einen Polyethylenglykolether der Formel I enthält,
R¹ - O - (CH₂CH₂O)ₙ - R² (I),
in der R¹ einen geradkettigen oder verzweigten Alkyl- oder Alkenylrest mit 8 bis 18 Kohlenstoffatomen, R² einen geradkettigen oder verzweigten Alkylrest mit 4 bis 8 Kohlenstoffatomen und n eine Zahl von 7 bis 12 bedeutet.

6. Verfahren nach Anspruch 5, dadurch gekennzeichnet, daß die Reinigungs- und Desinfektionsmittellösung in Schritt a) einen Polyethylenglykolether der Formel I in Anspruch 5 enthält, in der R¹ für ein aus einem Talgfettalkohol stammendes Gemisch aus Alkyl- und/oder Alkenylresten mit 12 bis 18 Kohlenstoffatomen und R² für einen n-Butylrest steht, während n eine Zahl von 9 bis 10 bedeutet.

7. Verfahren nach den Ansprüchen 1 bis 6, dadurch gekennzeichnet, daß die Reinigungs- und Desinfektionsmittellösung in Schritt a) einen Komplexbildner aus der Gruppe Alkalisalze der Nitrilotriessigsäure, Ethylendiaminotetraessigsäure, 1-Hydroxyethan-1,1-diphosphonsäure, Aminotris-(methylenphosphonsäure), Ethylendiaminotetrakis-(methylenphosphonsäure), Phosphonobutantricarbonsäure, Weinsäure, Citronensäure und Glukonsäure enthält.

8. Verfahren nach Anspruch 7, dadurch gekennzeichnet, daß die Reinigungs- und Desinfektionsmittellösung in Schritt a) als Komplexbildner Phosphonobutantricarbonsäure enthält.

9. Verfahren nach den Ansprüchen 1 bis 8, dadurch gekennzeichnet, daß die Reinigungs- und Desinfektionsmittellösung in Schritt a) Glutaraldehyd enthält.

10. Verfahren nach den Ansprüchen 1 bis 9, dadurch gekennzeichnet, daß man im Schritt a) die Endoskope der Einwirkung von Ultraschall aussetzt.

11. Verfahren nach den Ansprüchen 1 bis 10, dadurch gekennzeichnet, daß man die zu behandelnden Endoskope im Schritt c) mit Heißluft behandelt, die mit Hilfe eines Mikrofilters sterilisiert wurde.

## Claims

1. A process for the cleaning and disinfection of heat- and corrosion-sensitive medical instruments, particularly endoscopes, using aqueous cleaning and disinfectant solutions, characterized in that, in successive steps,
a) the surfaces to be treated are brought into contact with a detergent and disinfectant solution which contains
- at least one low-foaming nonionic surfactant,
- at least one proteolytic enzyme,
- at least one complexing agent,
- at least one aldehyde from the group consisting of formaldehyde and aliphatic dialdehydes containing 2 to 8 carbon atoms and
- optionally other standard detergent and disinfectant constituents
and which has a pH value of from 6 to 8, the cleaning solution is heated to 55 to 65°C, kept at that temperature for 1 to 15 minutes and then drained off,
b) the surfaces to be treated are rinsed at least twice with water, the water being heated to 55 to 65°C at least in the last wash cycle and
c) are dried with sterilized hot air at 40 to 60°C,
water having a hardness of 3 to 8°Gh (German hardness) being used in steps a) and b).

2. A process as claimed in claim 1, characterized in that the detergent and disinfectant solution in step a) contains
0.1 to 1.0 g/l low-foaming surfactant,
0.03 to 0.3 AU/l proteolytic enzyme,
0.02 to 0.3 g/l complexing agent and
0.5 to 5 g/l aldehyde
(AU = Anson Units).

3. A process as claimed in claims 1 and 2, characterized in that the detergent and disinfectant solution in step a) contains as low-foaming nonionic surfactant an alkylene oxide adduct of the type obtainable by addition of 3 to 30 mol ethylene oxide and/or propylene oxide onto aliphatic polyols containing 2 to 6 hydroxyl groups and 2 to 12 carbon atoms and onto fatty alcohols, fatty acids, fatty amines or alkylphenols each containing 8 to 18 carbon atoms, the terminal hydroxyl groups of these polyglycol ether derivatives optionally being etherified, esterified or acetalized.

4. A process as claimed in claims 1 to 3, characterized in that the detergent and disinfectant solution in step a) contains a low-foaming nonionic surfactant from the group consisting of adducts of 3 to 15 mol ethylene oxide with saturated and unsaturated C₈₋₁₈ fatty alcohols, adducts of 3 to 5 mol ethylene oxide and 3 to 6 mol propylene oxide with saturated and unsaturated C₈₋₁₈ fatty alcohols and ether derivatives of these fatty alcohol polyalkylene glycol ethers, in which the terminal hydroxyl groups are etherified with a straight-chain or branched, saturated aliphatic C₄₋₈ alcohol.

5. A process as claimed in claim 4, characterized in that the detergent and disinfectant solution in step a) contains as the low-foaming surfactant a polyethylene glycol ether corresponding to the following formula
R¹ - O - (CH₂CH₂O)ₙ - R² (I)
in which R¹ is a straight-chain or branched-chain C₈₋₁₈ alkyl or alkenyl radical, R² is a straight-chain or branched-chain C₄₋₈ alkyl radical and n is a number of 7 to 12.

6. A process as claimed in claim 5, characterized in that the detergent and disinfectant solution in step a) contains a polyethylene glycol ether corresponding to formula I in claim 5 in which R¹ is a mixture of C₁₂₋₁₈ alkyl and/or alkenyl radicals emanating from a tallow fatty alcohol and R² is an n-butyl radical while n is a number of 9 to 10.

7. A process as claimed in claims 1 to 6, characterized in that the detergent and disinfectant solution in step a) contains a complexing agent from the group consisting of alkali metal salts of nitrilotriacetic acid, ethylenediamine tetraacetic acid, 1-hydroxyethane-1,1-diphosphonic acid, aminotris-(methylenephosphonic acid), ethylenediamine tetrakis-(methylenephosphonic acid), phosphonobutane tricarboxylic acid, tartaric acid, citric acid and gluconic acid.

8. A process as claimed in claim 7, characterized in that the detergent and disinfectant solution in step a) contains phosphonobutane tricarboxylic acid as complexing agent.

9. A process as claimed in claims 1 to 8, characterized in that the detergent and disinfectant solution in step a) contains glutaraldehyde.

10. A process as claimed in claims 1 to 9, characterized in that the endoscopes are ultrasonicated in step a).

11. A process as claimed in claims 1 to 10, characterized in that the endoscopes to be treated are treated in step c) with hot air which has been sterilized by passage through a microfilter.

## Revendications

1. Procédé de nettoyage et de désinfection des équipements médicaux sensibles à la chaleur et à la corrosion, en particulier d'endoscopes, à l'aide de solutions aqueuses d'agents nettoyants et désinfectants, qui est caractérisé en ce que l'on
a) met successivement en contact les surfaces à traiter des endoscopes avec une solution d'agents nettoyants et désinfectants, qui renferme
- au moins un tensioactif non ionique à mousse freinée,
- au moins une enzyme protéolytique,
- au moins un agent complexant,
- au moins un aldéhyde faisant partie du groupe constitué du formaldéhyde et des dialdéhydes aliphatiques comportant 2 à 8 atomes de carbone et
- le cas échéant, d'autres constituants usuels des agents nettoyants et désinfectants
et qui possède un pH compris entre 6 et 8, en ce que l'on chauffe la solution de nettoyage entre 55 et 65 °C, la maintient à cette température pendant 1 à 15 minutes et l'élimine ensuite,
b) rince les surfaces à traiter au moins deux fois à l'eau, en chauffant l'eau au moins au cours du dernier rinçage entre 55 et 65 °C, et
c) sèche à l'air chaud stérilisé entre 40 et 60 °C,
en mettant en oeuvre au cours des étapes a) et b) de l'eau d'une dureté de 3 à 8 °d.

2. Procédé selon la revendication 1, caractérisé en ce que la solutions d'agents nettoyants et désinfectants mise en oeuvre dans l'étape a) renferme
0,1 à 1,0 g/l de tensioactif à mousse freinée,
0,03 à 0,3 AE/l d'enzyme protéolytique,
0,02 à 0,3 g/l d'agent complexant et
0,5 à 5 g/l d'aldéhyde
(AE = unités Anson).

3. Procédé selon les revendications 1 et 2, caractérisé en ce que la solution d'agents nettoyants et désinfectants mise en oeuvre dans l'étape a) renferme comme tensioactif non ionique à mousse freinée un adduit d'oxyde d'alkylène, qui peut être obtenu par addition de 3 à 30 moles d'oxyde d'éthylène et/ou d'oxyde de propylène à des polyols aliphatiques comportant 2 à 6 groupes hydroxyle et 2 à 12 atomes de carbone, ainsi qu'à des alcools gras, acides gras, amines grasses ou alkylphénols comportant 8 à 18 atomes de carbone dans chaque cas, les groupes hydroxyle en fin de chaîne de ces dérivés de polyglycoléther pouvant également être éthérifiés, estérifiés ou acétalisés.

4. Procédé selon les revendications 1 à 3, caractérisé en ce que la solution d'agents nettoyants et désinfectants mise en oeuvre dans l'étape a) renferme un tensioactif non ionique à mousse freinée faisant partie du groupe constitué des produits d'addition de 3 à 15 moles d'oxyde d'éthylène à des alcools gras saturés et insaturés comportant 8 à 18 atomes de carbone, des produits d'addition de 3 à 5 moles d'oxyde d'éthylène et de 3 à 6 moles d'oxyde de propylène à des alcools gras saturés et insaturés comportant 8 à 18 atomes de carbone, ainsi que des dérivés d'éther de ces alkylèneglycoléthers d'alcools gras, dans lesquels les groupes hydroxyle en fin de chaîne sont éthérifiés au moyen d'un alcool aliphatique saturé à chaîne droite ou ramifiée comportant 4 à 8 atomes de carbone.

5. Procédé selon la revendication 4, caractérisé en ce que la solution d'agents nettoyants et désinfectants mise en oeuvre dans l'étape a) renferme comme tensioactif à mousse freinée un polyéthylèneglycoléther de la formule I
R¹ - O (CH₂CH₂O)ₙ - R² (I),
dans laquelle R¹ représente un radical alkyle ou alcényle à chaîne droite ou ramifiée comportant 8 à 18 atomes de carbone, R₂ correspond à un radical alkyle à chaîne droite ou ramifiée comprenant 4 à 8 atomes de carbone et n est un nombre de 7 à 12.

6. Procédé selon la revendication 5, caractérisé en ce que la solution d'agents nettoyants et désinfectants mise en oeuvre dans l'étape a) renferme un polyéthylèneglycoléther de la formule I dans la revendication 5, dans laquelle R¹ représente un mélange de radicaux alkyle et/ou alcényle comportant 12 à 18 atomes de carbone, provenant d'un alcool gras de suif durci ou non durci, R² correspond à un radical n-butyle, et n est un nombre de 9 à 10.

7. Procédé selon les revendications 1 à 6, caractérisé en ce que la solution d'agents nettoyants et désinfectants mise en oeuvre dans l'étape a) renferme un agent complexant faisant partie du groupe constitué des sels de métaux alcalins des acides nitrilotriacétique, éthylènediamino-tétracétique, 1-hydroxyéthane-1,1-diphosphonique, aminotris-(méthylènephosphonique), éthylènediaminotétrakis-(méthylènephosphonique), phosphonobutanetricarboxylique, tartrique, citrique et gluconique.

8. Procédé selon la revendication 8, caractérisé en ce que la solution d'agents nettoyants et désinfectants mise en oeuvre dans l'étape a) renferme comme agent complexant, de l'acide phosphonobutanetricarboxylique.

9. Procédé selon les revendications 1 à 8, caractérisé en ce que la solution d'agents nettoyants et désinfectants mise en oeuvre dans l'étape a) renferme du glutaraldéhyde.

10. Procédé selon les revendications 1 à 9, caractérisé en ce que l'on expose l' endoscope à l'action d'ultrasons dans l'étape a).

11. Procédé selon les revendications 1 à 10, caractérisé en ce que l'on traite dans l'étape c) les endoscopes à traiter par de l'air chaud, qui a été stérilisé au moyen d'un microfiltre.
